# EUROPEAN PATENT APPLICATION

(11) **EP 3 485 807 A1**
(43) Date of publication of application: **22.05.2019**
(21) Application number: 17827442.9
(22) Date of filing: 29.06.2017
(51) Int. Cl.: A61B 5/0285, A61B 5/1455

(54) **MEASUREMENT DEVICE, MEASUREMENT METHOD, AND MEASUREMENT PROGRAM**

(30) Priority: 14.07.2016 JP 2016139565
(71) Applicant: KYOCERA Corporation, Kyoto 612-8501 (JP)
(72) Inventor: HIGUCHI Daisuke, Kyoto-shi Kyoto 612-8501 (JP)
(74) Representative: Viering, Jentschura & Partner mbB Patent- und Rechtsanwälte
(86) International application number: PCT/JP2017/024058
(87) International publication number: WO 2018/012312

(57) **Abstract**

A measurement apparatus includes an optical emitter, an optical detector, and a controller. The optical emitter emits irradiation light onto a region. The optical detector detects scattered light from the region, the scattered light corresponding to the irradiation light. The controller causes first irradiation light of a first intensity and second irradiation light of a second intensity lower than the first intensity to be emitted from the optical emitter. The controller detects a predetermined signal on the basis of first scattered light detected by the optical detector in accordance with the first irradiation light and second scattered light detected by the optical detector in accordance with the second irradiation light.

## Description

### CROSS-REFERENCE TO RELATED APPLICATION

The present application claims priority to and the benefit of Japanese Patent Application No. 2016-139565 filed July 14, 2016, the entire contents of which are incorporated herein by reference.

### TECHNICAL FIELD

The present disclosure relates to a measurement apparatus, a measurement method, and a measurement program.

### BACKGROUND

A known measurement apparatus includes a measurement unit that acquires biological information of a subject on the basis of scattered light resulting from light irradiated onto the subject. The information acquired by the measurement apparatus includes, for example, information pertaining to noise due to movement of the subject. To remove the information pertaining to noise, the measurement apparatus may include not only a measurement unit that acquires biological information of the subject but also a measurement unit that acquires only information pertaining to noise. For example, see patent literature (PTL) 1. An optical blood flow measurement apparatus that uses a plurality of different wavelengths of light is also known. For example, see PTL 2.

### CITATION LIST

### Patent Literature

PTL 1: JP2012-143316A
PTL 2: JP2013-150772A

### SUMMARY

A measurement apparatus according to an aspect includes an optical emitter that emits irradiation light onto a region. The measurement apparatus includes an optical detector that detects scattered light from the region, the scattered light corresponding to the irradiation light. The measurement apparatus includes a controller. The controller causes first irradiation light of a first intensity and second irradiation light of a second intensity lower than the first intensity to be emitted from the optical emitter at different timings. The controller detects a predetermined signal on the basis of first scattered light detected by the optical detector in accordance with the first irradiation light and second scattered light detected by the optical detector in accordance with the second irradiation light.

A measurement method according to an aspect includes emitting first irradiation light of a first intensity and second irradiation light of a second intensity lower than the first intensity onto a region at different timings. The measurement method includes detecting a predetermined signal on the basis of first scattered light detected in accordance with the first irradiation light and second scattered light detected in accordance with the second irradiation light.

A measurement program according to an aspect causes a computer to execute the step of emitting first irradiation light of a first intensity and second irradiation light of a second intensity lower than the first intensity onto a region at different timings. The measurement program causes the computer to execute the step of detecting a predetermined signal on the basis of first scattered light detected in accordance with the first irradiation light and second scattered light detected in accordance with the second irradiation light.

### BRIEF DESCRIPTION OF THE DRAWINGS

In the accompanying drawings:
FIG. 1 is a block diagram schematically illustrating an example configuration of a measurement apparatus according to an embodiment;
FIG. 2 illustrates an example of optical paths of irradiation light and scattered light;
FIG. 3 illustrates an example of optical paths of irradiation light and scattered light; and
FIG. 4 is a flowchart illustrating an example of the procedures of a measurement method according to an embodiment.

### DETAILED DESCRIPTION

When biological information of the subject is acquired, the accuracy of the biological information can be improved by the removal of noise.

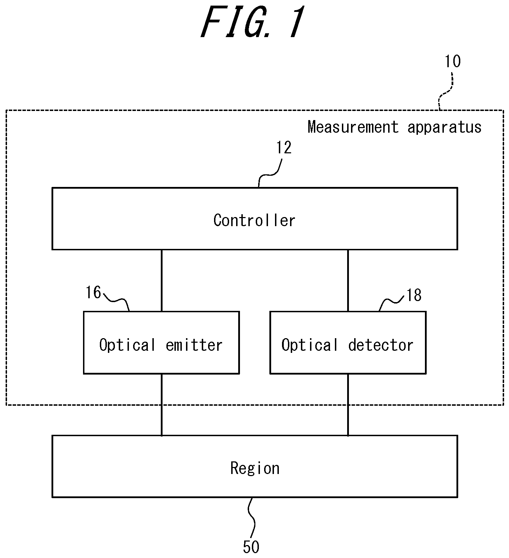

As illustrated in FIG. 1, a measurement apparatus 10 according to an embodiment includes a controller 12, an optical emitter 16, and an optical detector 18. The measurement apparatus 10 acquires biological information of the subject from a region 50 of the subject in a contact or non-contact manner.

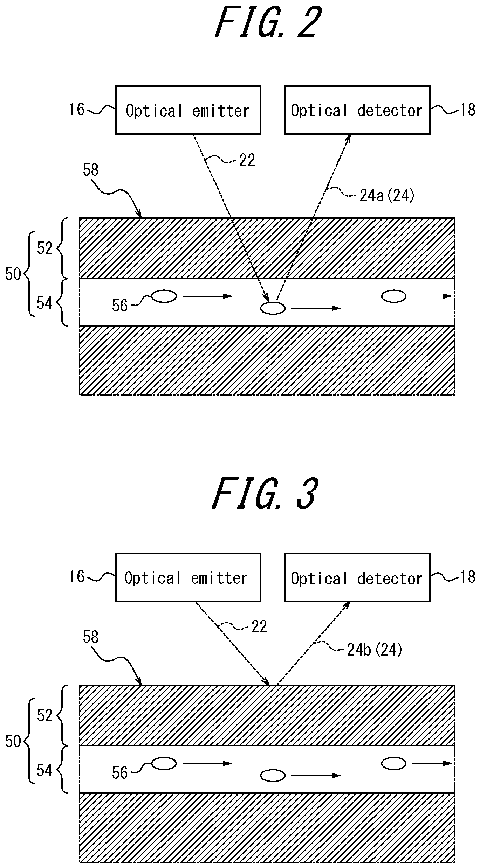

On the basis of control information from the controller 12, the optical emitter 16 emits irradiation light 22 onto the region 50 as illustrated in FIG. 2, for example. The optical emitter 16 includes a light-emitting element, such as a light-emitting diode (LED) or a laser diode (LD). The optical emitter 16 may include one light-emitting element or more than one light-emitting element. The optical emitter 16 may emit light of a predetermined wavelength. The optical emitter 16 may emit light of a plurality of wavelengths. The optical emitter 16 may emit light having wavelengths over a predetermined range.

The optical detector 18 detects the intensity of scattered light 24 from the region 50, the scattered light 24 corresponding to the irradiation light 22. The scattered light 24 from the region 50 can include biological information of the subject. The optical detector 18 outputs the detected result to the controller 12 as detection information. The optical detector 18 includes a light-receiving element, such as a photodiode (PD) or a phototransistor (PT). The optical detector 18 may include one light-receiving element or more than one light-receiving element. The optical detector 18 detects light of a predetermined wavelength. The optical detector 18 may detect light of a plurality of wavelengths. The optical detector 18 may detect light having wavelengths over a predetermined range.

The controller 12 outputs control information to the optical emitter 16 for causing the optical emitter 16 to emit the irradiation light 22. The controller 12 acquires the detection information from the optical detector 18. The controller 12 may be a processor for controlling the functional blocks or the entirety of the measurement apparatus 10. The controller 12 may be a central processing unit (CPU) or other processor that executes programs, such as a program prescribing control procedures of the measurement apparatus 10. The controller 12 may be a computer that includes a processor. The program may, for example, be stored on a storage medium. The controller 12 may include a storage medium. The controller 12 may be connected to an external storage medium. The controller 12 may work with a program to execute the below-described functions of the measurement apparatus 10.

The measurement apparatus 10 can use the Doppler effect to measure the blood flow. The measurement of blood flow based on the Doppler effect is also referred to as Doppler blood flowmetry. As illustrated in FIG. 2, the region 50 may include living tissue 52 and a blood vessel 54. The living tissue 52 is assumed to be on the body surface 58 side of the subject. The blood vessel 54 is assumed to be farther from the body surface 58 of the subject than the living tissue 52 is. The irradiation light 22 emitted from the optical emitter 16 to the region 50 may pass through the living tissue 52 and reach the blood vessel 54. The blood vessel 54 may be an artery, a vein, a capillary, or any combination thereof. The irradiation light 22 can be scattered by blood cells 56 in the blood flowing through the blood vessel 54. The scattered light 24 produced when the irradiation light 22 is scattered by the blood cells 56 is also referred to as blood cell scattered light 24a.

The frequency of the blood cell scattered light 24a is shifted from the frequency of the irradiation light 22 by the Doppler effect. The frequency shift between the frequency of the irradiation light 22 and the frequency of the blood cell scattered light 24a due to the Doppler effect is also referred to as a Doppler shift. The shifted frequency due to the Doppler shift is also referred to as the Doppler shift frequency. The Doppler shift frequency can be considered proportional to the moving speed of the blood cells 56 by use of an approximation calculation based on how the moving speed of the blood cells 56 is much slower than the speed of light.

As illustrated in FIG. 3, the irradiation light 22 emitted onto the region 50 from the optical emitter 16 may be scattered by the living tissue 52 or the body surface 58 of the subject before reaching the blood vessel 54. The scattered light 24 produced when the irradiation light 22 is scattered by the living tissue 52 or the body surface 58 is also referred to as living tissue scattered light 24b. The frequency of the living tissue scattered light 24b is assumed to be unaltered by the Doppler effect and to be the same as the frequency of the irradiation light 22.

The optical detector 18 detects combined scattered light in which the blood cell scattered light 24a and the living tissue scattered light 24b are combined. The combined scattered light can become a beat signal. The amplitude of the beat signal changes in accordance with the beat frequency. The beat frequency can correspond to the Doppler shift frequency. The Doppler shift frequency is the difference between the frequency of the blood cell scattered light 24a and the frequency of the living tissue scattered light 24b. In other words, the amplitude of the combined scattered light can change in accordance with the Doppler shift frequency. The optical detector 18 detects the intensity of the combined scattered light. The intensity of the combined scattered light is proportional to the square of the amplitude of the combined scattered light. Consequently, the frequency of a change in intensity of the combined scattered light is twice the frequency of a change in the amplitude of the combined scattered light. The intensity of the combined scattered light can change in accordance with a frequency that is twice the Doppler shift frequency. The optical detector 18 may detect the intensity of the combined scattered light continuously or at discrete points in time and may output a waveform representing the change over time in the intensity of the combined scattered light as detection information. The detection information output by the optical detector 18 includes information pertaining to the blood flow of the subject. The information pertaining to the blood flow of the subject is also referred to as blood flow information.

The controller 12 acquires the detection information from the optical detector 18. The controller 12 analyzes the frequency spectrum of the detection information. This detection information is a waveform representing the change over time in the intensity of the combined scattered light. The controller 12 calculates the power spectrum of the combined scattered light. The power spectrum may represent the waveform representing the change over time in the intensity of light as a function of frequency.

In the power spectrum of the combined scattered light, the frequency corresponds to the moving speed of the blood cells 56. The intensity of each frequency corresponds to the number of blood cells 56. The power spectrum of the combined scattered light represents the number of blood cells 56 at each moving speed of the blood cells 56.

The controller 12 integrates the product of the frequency and the intensity at each frequency over a predetermined frequency band. In other words, the controller 12 integrates the product of a parameter corresponding to the moving speed of the blood cells 56 and a parameter corresponding to the number of blood cells 56 having each moving speed. This allows the controller 12 to calculate a parameter corresponding to the blood flow. The parameter corresponding to the blood flow can be converted into the blood flow on the basis of correction data. The correction data can, for example, be calculated on the basis of the blood flow acquired by another measurement method. The parameter corresponding to the blood flow may be standardized using the total intensity of light detected by the optical detector 18. This allows the blood flow to be calculated at higher accuracy even when the intensity of the irradiation light 22 or the optical detection sensitivity of the optical detector 18 varies.

The detection information output by the optical detector 18 can include not only blood flow information but also information pertaining to noise. The information pertaining to noise is also referred to as noise information. Noise can, for example, be produced by a change in the relative positions between the measurement apparatus 10 and the region 50 due to movement by the subject. The noise produced by movement of the subject is also referred to as body movement noise. By calculating the difference between detection information that includes blood flow information and noise information and detection information that only includes noise information, the controller 12 can remove the noise information from the detection information to acquire the blood flow information alone.

When the irradiation light 22 is assumed not to reach the blood vessel 54 and to be entirely scattered by the living tissue 52, the detection information based on detection of the scattered light 24 does not include blood flow information. By appropriately setting the intensity of the irradiation light 22, the controller 12 can make it difficult for the irradiation light 22 to reach the blood vessel 54.

The irradiation light 22 can penetrate more deeply into the region 50 as the intensity of the irradiation light 22 is higher. The intensity of the irradiation light 22 at which at least a portion of the irradiation light 22 can reach the blood vessel 54 is designated as a first intensity. In contrast, the intensity of the irradiation light 22 at which none of the irradiation light 22 reaches the blood vessel 54 is designated as a second intensity. The second intensity can be set lower than the first intensity. A predetermined intensity may be set so that the first intensity is equal to or greater than the predetermined intensity and the second intensity is less than the predetermined intensity. The second intensity may be set so that all of the irradiation light 22 is scattered by the surface of the living tissue 52.

The measurement apparatus 10 emits first irradiation light having the first intensity and second irradiation light having the second intensity onto the region 50 from the optical emitter 16. The measurement apparatus 10 emits the first irradiation light and the second irradiation light at different timings. The timing at which the first irradiation light is emitted is also referred to as the first timing. The timing at which the second irradiation light is emitted is also referred to as the second timing. The first timing may be earlier or later than the second timing.

The measurement apparatus 10 detects first scattered light with the optical detector 18. The first scattered light is produced when the first irradiation light is scattered by the region 50. The measurement apparatus 10 acquires first detection information from the first scattered light. The first scattered light is combined scattered light in which the blood cell scattered light 24a and the living tissue scattered light 24b are combined. The first detection information can include blood flow information and noise information.

The measurement apparatus 10 detects second scattered light with the optical detector 18. The second scattered light is produced when the second irradiation light is scattered by the region 50. The measurement apparatus 10 acquires second detection information from the second scattered light. The second scattered light can include no blood cell scattered light 24a, or less blood cell scattered light 24a than the blood cell scattered light 24a included in the first scattered light, and can include the living tissue scattered light 24b. The second detection information can include no blood flow information, or less blood flow information than the blood flow information included in the first detection information, and can include noise information.

The measurement apparatus 10 calculates the difference between the first detection information and the second detection information. On the basis of this difference, the measurement apparatus 10 can acquire blood flow information from which the noise information is removed. The measurement apparatus 10 may acquire the blood flow information from which the noise information is removed on the basis of the difference between the intensity of the combined scattered light based on the first irradiation light and the intensity of the combined scattered light based on the second irradiation light. The measurement apparatus 10 may acquire the blood flow information from which the noise information is removed on the basis of the difference between the intensity of the power spectrum of the combined scattered light based on the first irradiation light and the intensity of the power spectrum of the combined scattered light based on the second irradiation light.

The measurement apparatus 10 may emit the first irradiation light throughout a first irradiation time. The measurement apparatus 10 may emit the second irradiation light throughout a second irradiation time. The first irradiation time and the second irradiation time can be set appropriately. The first irradiation time may be set so that the waveform representing the change over time in the intensity of the combined scattered light is acquired with a sufficient length for frequency spectrum analysis. The first irradiation time may, for example, be set to 50 ms or less but is not limited to this value. The second irradiation time may be set in the same way or a similar way as the first irradiation time. The second irradiation time may be set so that noise information due to movement of the subject is acquired. The second irradiation time may, for example, be set to 50 ms or less but is not limited to this value.

When the first irradiation time and the second irradiation time are each set to 25 ms, the measurement apparatus 10 can, in 50 ms, acquire the blood flow information from which the noise information is removed. The subject rarely changes movements within a time period of approximately several tens of milliseconds when performing normal activities or light exercise. Consequently, the measurement apparatus 10 can acquire the blood flow information in most cases while the subject is not changing movements. The noise information included in the first detection information and the second detection information that are acquired while the subject is making the same movement can be considered the same information. The measurement apparatus 10 can remove the noise information even when the first irradiation light and the second irradiation light are emitted at different timings.

An apparatus according to a comparative example includes a first optical emitter that emits first irradiation light having a first intensity and a second optical emitter that emits second irradiation light having a second intensity. The apparatus according to the comparative example includes a first optical detector corresponding to the first optical emitter and a second optical detector corresponding to the second optical emitter. The first optical detector can detect first scattered light, and the second optical detector can detect second scattered light.

The apparatus according to the comparative example causes the first irradiation light from the first optical emitter and the second irradiation light from the second optical emitter to be emitted simultaneously. The apparatus according to the comparative example detects the first scattered light with the first optical detector and the second scattered light with the second optical detector to acquire first detection information and second detection information.

In the comparative example, the first irradiation light and the second irradiation light are irradiated onto different regions 50. In other words, the first scattered light and the second scattered light are light scattered by different regions 50. The first detection information and the second detection information include noise information for different regions 50. The noise information for different regions 50 may differ due to the conditions in which the subject wears the apparatus, the effect of movement of the wearing portion of the apparatus, or the like. Consequently, the accuracy with which noise is removed by calculating the difference between the first detection information and the second detection information may lower. Furthermore, it is difficult to reduce the size and costs of an apparatus that includes two or more measurement units.

The measurement apparatus 10 according to the present embodiment can calculate blood flow information from which noise information is removed by using noise information acquired from the same region 50. This allows the measurement apparatus 10 to remove noise more accurately and to be configured with fewer components than the apparatus according to the comparative example.

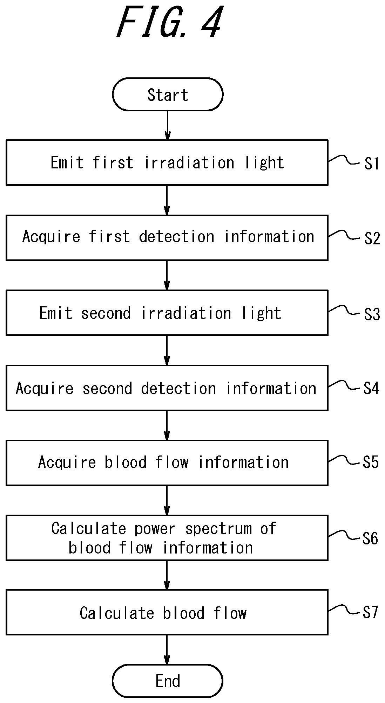

The measurement apparatus 10 according to the present embodiment may execute a measurement method according to the procedures illustrated in FIG. 4.

The controller 12 emits the first irradiation light from the optical emitter 16 onto the region 50 (step S1). The controller 12 detects the first scattered light from the region 50 with the optical detector 18 and acquires the first detection information (step S2). The first detection information can include blood flow information and noise information.

The controller 12 emits the second irradiation light from the optical emitter 16 onto the region 50 (step S3). The controller 12 detects the second scattered light from the region 50 with the optical detector 18 and acquires the second detection information (step S4). The second detection information can include no blood flow information, or less blood flow information than the blood flow information included in the first detection information, and can include noise information.

The controller 12 calculates the difference between the first detection information and the second detection information to acquire blood flow information from which the noise information is removed (step S5).

The controller 12 calculates the power spectrum of the blood flow information (step S6). The controller 12 analyzes the frequency spectrum of the waveform, acquired as the blood flow information, representing the change over time in the intensity of the combined scattered light.

The controller 12 calculates the blood flow on the basis of the power spectrum of the blood flow information (step S7). In the power spectrum of the blood flow information, the frequency corresponds to the speed of the blood cells 56, and the intensity of each frequency corresponds to the number of blood cells 56. The controller 12 can integrate the product of the frequency and the intensity at each frequency over a predetermined frequency band to calculate a parameter corresponding to the blood flow. The controller 12 may calculate the blood flow on the basis of the parameter corresponding to the blood flow and correction data. A non-limiting example of the correction data is the blood flow acquired by another measurement method.

The controller 12 ends the procedures of the flowchart in FIG. 4 after step S7. The controller 12 may return to step S1 and repeat the procedures of the flowchart in FIG. 4 after step S7.

The difference between the power spectrum of the first detection information and the power spectrum of the second detection information can be the same as the power spectrum of the blood flow information. Before calculating the power spectrum of the blood flow information, the controller 12 may calculate the power spectra of the first detection information and the second detection information. The controller 12 may then calculate the difference between the power spectrum of the first detection information and the power spectrum of the second detection information. In this case, the controller 12 may calculate the power spectrum of the first detection information after executing step S2. The controller 12 may calculate the power spectrum of the second detection information after executing step S4.

Before executing the procedures of step S1 and step S2, the controller 12 may execute the procedures of step S3 and step S4.

In addition to calculating a parameter pertaining to the blood flow of the subject or calculating the blood flow of the subject, the measurement apparatus 10 may also measure the pulse wave of the subject. The pulse wave of the subject can be represented as a waveform representing the change over time in a parameter pertaining to the blood flow of the subject or the change over time in the blood flow of the subject.

The measurement apparatus 10 can calculate a parameter pertaining to the blood flow of the subject, or calculate the blood flow, on the basis of a pair of the first scattered light and the second scattered light. The blood flow varies during one heartbeat. The measurement apparatus 10 can measure the pulse wave by measuring the change over time in a parameter pertaining to the blood flow, or the change over time in the blood flow, continuously or at discrete points in time.

The measurement apparatus 10 can be configured to be capable of measuring the blood flow of the subject within a predetermined length of time. The predetermined length of time can be set on the basis of the first irradiation time and the second irradiation time of the optical emitter 16. The predetermined length of time may, for example, be set to 100 ms or less but is not limited to this value.

When the measurement apparatus 10 is capable of measuring the blood flow of the subject in 50 ms, 20 blood flow data points are measured in one second. If the subject's heart rate is one beat per second, then one cycle of the pulse wave can be represented as a waveform based on 20 blood flow data points. As the predetermined length of time is shorter, the number of blood flow data points representing one cycle of the pulse wave is higher. As the number of blood flow data points representing one cycle of the pulse wave is higher, the pulse wave can be measured with greater accuracy. The predetermined length of time may be set within a range allowing measurement of enough blood flow data points to represent one cycle of the pulse wave.

Configurations according to the present disclosure are not limited to the above embodiments, and a variety of modifications and changes are possible. For example, the functions and the like included in the various components may be reordered in any logically consistent way. Furthermore, components may be combined into one or divided.

For example, the measurement apparatus 10 may measure other biological information of the subject in addition to the blood flow or the pulse wave of the subject.

### REFERENCE SIGNS LIST

- 10: Measurement apparatus
- 12: Controller
- 16: Optical emitter
- 18: Optical detector
- 22: Irradiation light
- 24: Scattered light
- 24a: Blood cell scattered light
- 24b: Living tissue scattered light
- 50: Region
- 52: Living tissue
- 54: Blood vessel
- 56: Blood cell
- 58: Body surface

## Claims

1. A measurement apparatus comprising:
an optical emitter configured to emit irradiation light onto a region of a subject;
an optical detector configured to detect scattered light from the region, the scattered light corresponding to the irradiation light; and
a controller; wherein
the controller is configured to
cause first irradiation light of a first intensity and second irradiation light of a second intensity lower than the first intensity to be emitted from the optical emitter at different timings; and
detect a predetermined signal on the basis of first scattered light detected by the optical detector in accordance with the first irradiation light and second scattered light detected by the optical detector in accordance with the second irradiation light.

2. The measurement apparatus of claim 1, wherein the controller is configured to detect the predetermined signal on the basis of a difference between detection information of the first scattered light and detection information of the second scattered light.

3. The measurement apparatus of claim 1, wherein
the region includes living tissue of the subject and a blood vessel farther from a body surface than the living tissue is;
the first irradiation light is scattered by the living tissue and by blood flowing in the blood vessel;
the second irradiation light is scattered by the living tissue; and
scattered light from the blood has a frequency shifted from a frequency of the first irradiation light by the Doppler effect.

4. The measurement apparatus of claim 2, wherein
the region includes living tissue of the subject and a blood vessel farther from a body surface than the living tissue is;
the first irradiation light is scattered by the living tissue and by blood flowing in the blood vessel;
the second irradiation light is scattered by the living tissue; and
scattered light from the blood has a frequency shifted from a frequency of the first irradiation light by the Doppler effect.

5. A measurement method comprising:
emitting first irradiation light of a first intensity and second irradiation light of a second intensity lower than the first intensity onto a region at different timings; and
detecting a predetermined signal on the basis of first scattered light detected in accordance with the first irradiation light and second scattered light detected in accordance with the second irradiation light.

6. The measurement method of claim 5, further comprising detecting the predetermined signal on the basis of a difference between detection information of the first scattered light and detection information of the second scattered light.

7. The measurement method of claim 5, wherein
the region includes living tissue of a subject and a blood vessel farther from a body surface than the living tissue is;
the first irradiation light is scattered by the living tissue and by blood flowing in the blood vessel;
the second irradiation light is scattered by the living tissue; and
scattered light from the blood has a frequency shifted from a frequency of the first irradiation light by the Doppler effect.

8. The measurement method of claim 6, wherein
the region includes living tissue of a subject and a blood vessel farther from a body surface than the living tissue is;
the first irradiation light is scattered by the living tissue and by blood flowing in the blood vessel;
the second irradiation light is scattered by the living tissue; and
scattered light from the blood has a frequency shifted from a frequency of the first irradiation light by the Doppler effect.

9. A measurement program causing a computer to execute the steps of:
emitting first irradiation light of a first intensity and second irradiation light of a second intensity lower than the first intensity onto a region at different timings; and
detecting a predetermined signal on the basis of first scattered light detected in accordance with the first irradiation light and second scattered light detected in accordance with the second irradiation light.

10. The measurement program of claim 9, further comprising causing the computer to execute the step of detecting the predetermined signal on the basis of a difference between detection information of the first scattered light and detection information of the second scattered light.

11. The measurement program of claim 9, wherein
the region includes living tissue of a subject and a blood vessel farther from a body surface than the living tissue is;
the first irradiation light is scattered by the living tissue and by blood flowing in the blood vessel;
the second irradiation light is scattered by the living tissue; and
scattered light from the blood has a frequency shifted from a frequency of the first irradiation light by the Doppler effect.

12. The measurement program of claim 10, wherein
the region includes living tissue of a subject and a blood vessel farther from a body surface than the living tissue is;
the first irradiation light is scattered by the living tissue and by blood flowing in the blood vessel;
the second irradiation light is scattered by the living tissue; and
scattered light from the blood has a frequency shifted from a frequency of the first irradiation light by the Doppler effect.
